Europäisches Patentamt

European Patent Office

Office européen des brevets

(11) Publication number: **0 088 738**
**B1**

# (12) EUROPEAN PATENT SPECIFICATION

(45) Date of publication of patent specification: **08.02.89**

(21) Application number: **83850051.0**

(22) Date of filing: **04.03.83**

(51) Int. Cl.⁴: **A 61 F 13/16,** A 41 B 13/02, A 61 F 5/44

(54) **A method of manufacturing a fluid absorbing product.**

(30) Priority: **10.03.82 SE 8201489**

(43) Date of publication of application:
**14.09.83 Bulletin 83/37**

(45) Publication of the grant of the patent:
**08.02.89 Bulletin 89/06**

(84) Designated Contracting States:
**BE DE FR GB LU NL**

(56) References cited:
**DE-A-2 546 209**
**DE-A-2 853 243**
**DE-A-2 917 181**
**GB-A-2 042 901**

(73) Proprietor: **AB Akerlund & Rausing**
**Fack 1702**
**S-221 01 Lund (SE)**

(72) Inventor: **Fransson, Per**
**Trindyxvägen 9**
**S-291 65 Kristianstad (SE)**
Inventor: **Ekstrand, Stig**
**Envigsgränden 10**
**S-223 75 Lund (SE)**

(74) Representative: **Graudums, Valdis et al**
**Albihn West AB Stora Nygatan 15**
**S-411 08 Göteborg (SE)**

Courier Press, Leamington Spa, England.

**Description**

The present invention relates to a method of manufacturing a fluid absorbing product. In the most simple form thereof a fluid absorbing product may be regarded as a piece of equipment that allows inflow of a fluid to an absorbing core via a first side or portion thereof, but prevents penetration for the rest.

An apparatus for manufacturing such fluid absorbing products is claimed in our divisional application 86 202 068.2 (EP—A—0 231 536).

The inflow of a fluid for instance may take place through a layer of so called "fiber cloth" (non-woven) which also might possess a certain absorption capacity. A high liquid absorption is obtained by cellulose based fibres (cellulose, cotton, rayon), while synthetic fibres as polypropylene and polyester have a very low absorption capacity. As far as the pliability and handling of the non-woven material are concerned the fibre cloth material does not induce any problems in lines for manufacturing napkins, sanitary napkins, baby napkins, brief shields, sterile dressings, bandage material, etc. For instance, the material may be manufactured with a surface weight as low as 10 g/m² and still have an acceptable strength.

In addition to the necessary characteristics of allowing passage of fluid to the absorbing core, the fibre cloth material also acts to maintain the core in a proper position. Generally, the core consists of defibrated cellulose (defibrate) and additionally might include inserts of "super absorbing" material.

In order to give the fluid absorbing product penetration preventing characteristics — a liquid leakage barrier — in the regions thereof where absorption is not desired, previously it simply was inserted a loose film of plastics, for instance a polyethylene film, between the core and the carrier material. In certain cases the need for measures against leakage even was neglected.

A more sophisticated manufacturing method is disclosed in GB patent application No. 2 042 901. Said method includes the following steps:
a number of parallel fluid-impermeable layers for instance of thermoplastics material are applied to a web of a carrier material to form a laminated material, the web is cut length-wise generally along the centre line of each fluid-impermeable layer so as to produce a number of individual cut webs, the cut web is supplied with fluid absorbing core material for forming the absorbing core of the product and the cut web of the carrier material is folded around the core such that two coated areas of the cut web cover a portion of a broad side of the core and the narrow sides of the core such that each coated area extends a distance into the fluid absorbing broad side of the core. The laminate material of the wrappers for the absorbing cores are formed by utilizing for instance lamination techniques or extrusion.

According to the present invention it has been realized that the extrusion type of method may be improved considerably by implementing a specific extrusion technique when manufacturing fluid absorbing products.

The object of the present invention, therefore, is to improve the known technique and offer a more advantageous alternative.

The invention provides, in the broadest sense thereof, a method of manufacturing a fluid absorbing product comprising a fluid absorbing core and a fluid penetration preventing layer on a portion thereof, wherein a single web of a carrier material, for instance a non-woven fibre cloth material, is extrusion coated by a thermoplastics material, and the web is supplied with a fluid absorbing core material for forming the fluid absorbing core of the product. The method is characterized in that the extrusion process is a polyethylene extrusion strip coating process, in which a plurality of extruded strips are formed on the web, the polyethylene extrusion strip coating process inherently resulting a a thickening of the polyethylene layers in the edge regions of the extruded strips, that said extrusion strip coated web is cut length-wise generally along the centre line of each extruded strip so as to produce a plurality of cut webs, and that the cut web of the carrier material is folded around the core such that two coated areas of the cut web each cover a portion of a broad side of the core and at least a portion of a respective one of the narrow sides of the core, and such that each coated area extends with said thickened edge region of the polyethylene layer a distance into the fluid absorbing broad side of the core for defining the longitudinal marginal edges of a window for permitting fluid penetration into the fluid absorbing core.

Some embodiments of the invention will now be described with reference to the accompanying drawings, where

Figure 1 schematically shows a line for the manufacture of fluid absorbing products according to the invention, for instance napkins, brief shields, etc.,

Figure 2 in partial view shows a strip coated carrier material web,

Figure 3 shows the web in Figure 2 cut longitudinally and together with an absorbing core,

Figure 4 shows the arrangement in Figure 3 in section, and

Figure 5 in section shows the core according to Figure 4 with the carrier material applied around the core.

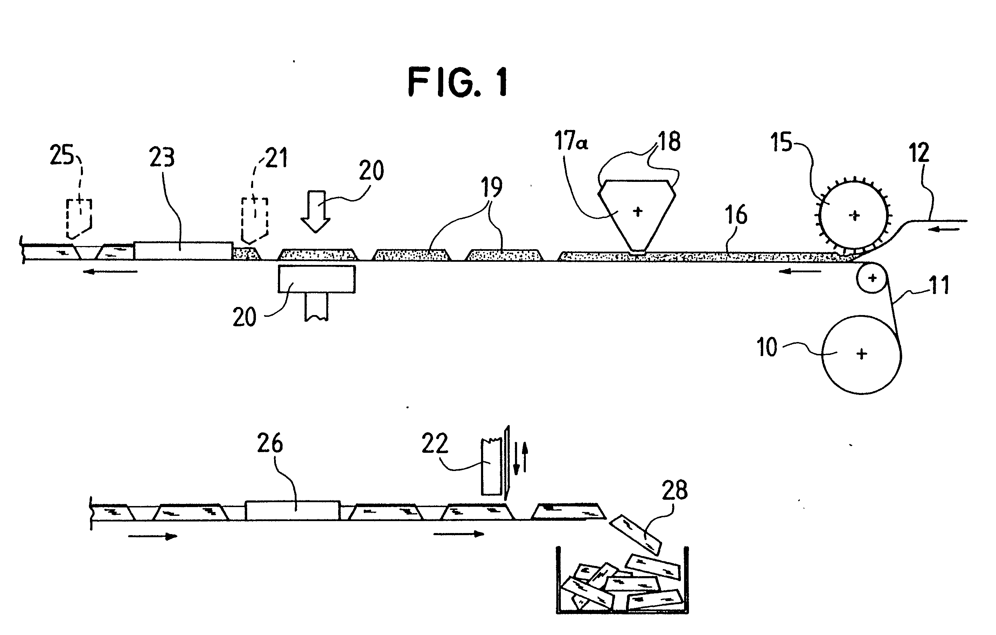

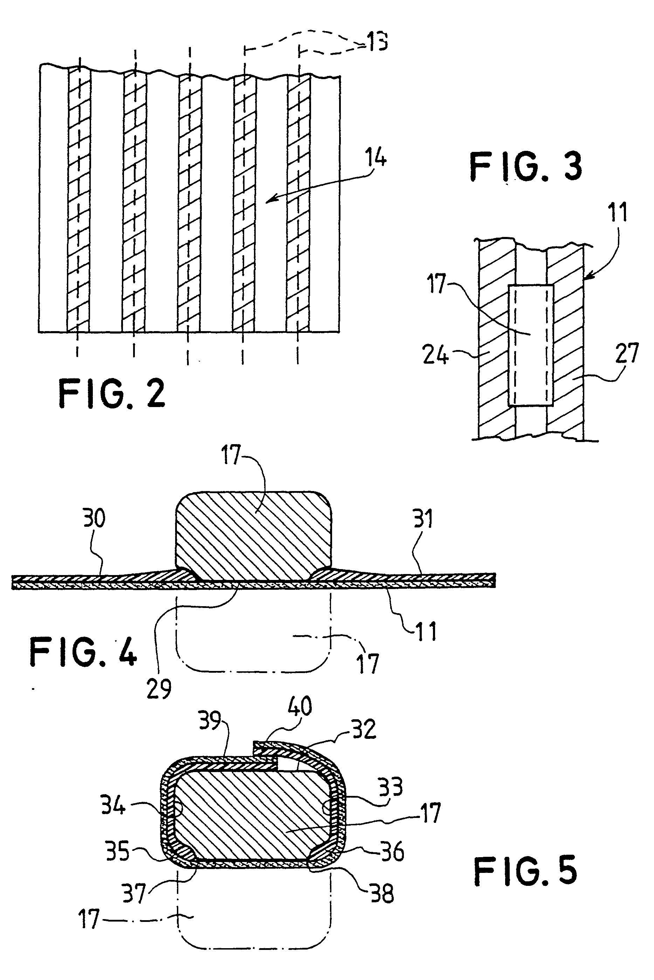

The reference numeral 10 in Figure 1 indicates a supply roller of carrier material. Said material consists of strip coated fibre cloth material (non-woven material) which has been extrusion strip coated by polyethylene and therafter cut length-wise into the desired width. The length-wise cutting is carried out along the broken lines 13 in Figure 2 in an extrusion strip coated, wide material web 14. This gives for instance an addition of thickness of the plastics material in strategically important positions, if the cut material 11 is placed as in Figures 3 to 5. The extrusion coating, namely, inherently implies thickening of the outer edges of the strips.

A web 12 of absorbing core material (fluff material), for instance of the defibrated cellulose type, is fed through a chopper 15 to the upper side of the cut web 11 where the material is spread out as a string 16 of sufficient thickness for the desired absorption capacity. A cross-wise discontinuity for allowing an efficient cross-wise seal is obtained in the web 16 by a rotating multi-orifice suction device 17a, in the present case comprising three suction orifices 18, one at each corner thereof. By giving the suction device a suitable rotation speed there are obtained individually separated core pieces 19 at the output side thereof.

Said pieces 19 are attached by suction to the pores of the cut web 11 in a suction/pressure station 20. The arrow in Figure 1 represents a pressure function and the box under the web is a suction box.

After the station 20, there eventually follows a first binding agent applicator 21, for instance a hot melt applicator, for applying a binding agent for the cross-sealing operation in an end station 22.

A first folding bar 23, for folding one 24 (Figure 3) of the side portions of the cut web 11 protruding from the absorption core 17, is arranged after the suction/pressure device and applicator 21. If required in certain cases to apply a specific binding agent in order to obtain sealing longitudinally, there is arranged a further hot melt applicator 25.

Thereafter follows a further folding station 26 for folding inwards and sealing of the other protruding edge portion 27 of the cut web 11 against the already folded-in first portion 24.

Finally, after this sealing, cross-wise sealing and cutting to finished products 28 is carried out in the end station 22.

Figures 3 to 5 show the absorbing product according to the invention, for instance a sanitary towel, brief shield, etc. In this case the absorbing core 17 is placed such that one broad side 29 thereof with edge portions extend into the strip coated regions 30, 31 which here broadly coincide with the extension of the edge portions 24, 27. This implies that after the folding of the end portions to the position according to Figure 5, the coated region of the product will cover completely the upper side 32 of the core, the two longitudinal narrow sides 33, 34 and will extend around the corners 35, 36 to positions 37, 38 a distance inwards the fluid penetrating broad side 29 of the absorbing body. This had turned out to be an excellent quarantee against leakage of liquid laterally out from the body, especially as the plastics layer is thicker in the regions 37, 38. If the product for instance is a brief shield, this implies that practically all lateral leakage is prevented independently of displacing forces acting on the shield. In this connection it may be mentioned that the actual brief shield is attached to the brief in a known manner with the upper side 39 of the carrier material against the brief.

The longitudinal seal 40 shown in Figure 5 might be a dot-wise or string-wise hot melt seal, alternatively the sealing may be accomplished by directly using the thermoplastics 30, 31.

If the carrier material is of the absorbing type the core 17 might advantageously be placed against the "uncoated" side of the carrier material (c.f. Figures 4 and 5).

## Claim

A method of manufacturing a fluid absorbing product comprising a fluid absorbing core (17) and a fluid penetration preventing layer (30, 31) on a portion thereof, wherein a single web (14) of a carrier material, for instance a non-woven fibre cloth material, is extrusion coated by a thermoplastics material, and the web is supplied with a fluid absorbing core material (16) for forming the fluid absorbing core (17) of the product, characterized in that the extrusion process is a polyethylene extrusion strip coating process, in which a plurality of extruded strips are formed on the web (14), the polyethylene extrusion strip coating process inherently resulting in a thickening of the polyethylene layers in the edge regions (37, 38) of the extruded strips, that said extrusion strip coated web (14) is cut length-wise generally along the centre line of each extruded strip so as to produce a plurality of cut webs (11), and that the cut web of the carrier material is folded around the core (17) such that two coated areas (30, 31) of the cut web (11) each cover a portion of a broad side (29) of the core (17) and at least a portion of a respective one of the narrow sides (33, 34) of the core, and such that each coated area (30, 31) extends with said thickened edge region (37, 38) of the polyethylene layer a distance into the fluid absorbing broad side (29) of the core for defining the longitudinal marginal edges of a window for permitting fluid penetration into the fluid absorbing core (17).

## Patentanspruch

Verfahren zur Herstellung eines Fließmittel absorbierenden Produktes mit einem Fließmittel absorbierenden Kern (17) und einer eine Fließmitteldurchdringung verhindernden Schicht (30, 31) auf einem Teil desselben, bei dem eine einzelne Bahn (14) eines Trägermaterials, wie beispielsweise ein nichtgewebtes Faserstoffmaterial, durch Extrudieren mit einem thermoplastischen Material beschichtet und die Bahn mit einem Fließmittel absorbierenden Kernmateiral (16) zur Bildung des Fließmittel absorbierenden Kernes (17) des Produktes versehen wird, dadurch gekennzeichnet, daß das Extrudierverfahren ein Polyethylenextrudier-Streifenbeschichtungsverfahren ist, in welchem auf der Bahn (14) mehrere extrudierte Streifen gebildet werden, wobei das Polyethyleneextrudier-Streifenbeschichtungsverfahren zu einer Verdickung der Polyethylenschichten in den Kantenbereichen (37, 38) der extrudierten Streifen führt, daß die durch Extrudieren mit Streifen beschichtete Bahn (14) in Längsrichtung allgemein entlang der Mittellinie eines jeden extrudierten Streifens derart geschnitten wird, daß mehrere geschnittene Bahnen (11) erzeugt werden, und daß die geschnittene Bahn (11) des Trägermaterials um den Kern (17) derart gefaltet wird, daß zwei beschichtete Bereiche (30, 31) der geschnittenen Bahn (11) jeweils einen Teil einer Breitseite (29) des Kerns (17) und wenigstens einen Teil einer einzelnen der Schmalseiten (33, 34) des Kerns bedecken und daß jeder überzogene Bereich (30, 31) sich mit dem verdickten Kantenbereich (37, 38) der Polyethylenschicht über einen Abstand in die Fließmittel absorbierende Breitseite (29) des Kerns erstreckt, um die Längsränder eines Fensters zu definieren, um so Fließmitteleindringung in den Fließmittel absorbierenden Kern (17) zu gestatten.

**Revendiction**

Procédé de fabrication d'un produit d'absorption de fluide comprenant un noyau d'absorption de fluide (17) et une couche d'empêchement de pénétration de fluide (30, 31) sur une partie de celui-ci, dans lequel une simple pièce (14) de matière de support, par exemple une matière en tissu fibreuse non tissée, est recouverte par extrusion par une matière thermoplastique, la pièce étant pourvue d'un matière de noyau d'absorption de fluide (16) pour former le noyau d'absorption de fluide (17) du produit, caractérisé en ce que le processs d'extrusion est un processus d'application de bandes par extrusion de polyéthylène, une série de bandes extrudées étant formées sur la pièce (14), le processus d'application de bandes par extrusion de polyéthylène conduisant intrinsèquement à un épaississement des couches de polyéthylène dans les zones marginales (37, 38) des bandes extrudées, en ce que la pièce recouverte de bandes d'extrusion (14) est coupé longitudinalement généralement le long de l'axe central de chaque bande extrudée de manière à produire une série de bandes coupées (11), et en ce que la pièce coupée (11) de la matière de support est repliée autour du noyau (17) de manière à ce que deux zones couvertes (30, 31) de la pièce coupée (11) recouvrent chacune une partie d'une face étendue (29) du noyau (17) et au moins une partie d'une des faces étroites (33, 34) respective du noyau, de manière à ce que chaque zone recouverte (30, 31) s'étende avec la zone marginale épaissie (37, 38) de la couche de polyéthylène sur une certaine distance sur la face étendue d'absorption de fluide (29) du noyau pour définir les bords marginaux longitudinaux d'une fenêtre pour permettre la pénétration de fluide dans le noyau d'absorption de fluide (17).

# FIG. 1

FIG. 2

FIG. 3

FIG. 4

FIG. 5